(19) Europäisches Patentamt / European Patent Office / Office européen des brevets

(11) EP 2 448 548 B1

(12) **EUROPEAN PATENT SPECIFICATION**

(45) Date of publication and mention of the grant of the patent:
**01.03.2017 Bulletin 2017/09**

(21) Application number: **10737210.4**

(22) Date of filing: **22.07.2010**

(51) Int Cl.:
***A61K 8/34*** *(2006.01)* ***A61K 8/37*** *(2006.01)*
***A61K 8/44*** *(2006.01)* ***A61K 8/63*** *(2006.01)*
***A61K 8/67*** *(2006.01)* ***A61K 8/97*** *(2017.01)*
***A61Q 19/02*** *(2006.01)*

(86) International application number:
**PCT/US2010/042846**

(87) International publication number:
**WO 2012/011905 (26.01.2012 Gazette 2012/04)**

(54) **METHOD FOR IMPROVING THE APPEARANCE OF HYPERPIGMENTED SPOT(S) USING AN EXTRACT OF LAMINARIA SACCHARINA**

VERFAHREN ZUR VERBESSERUNG DES AUSEHENS VON HYPERPIGMENTIERTEN FLECKEN MITTELS EINES EXTRAKTS VON LAMINARIA SACCHARINA

PROCÉDÉ POUR AMÉLIORER L'APPARENCE DES TÂCHES HYPERPIGMENTÉES UTILISANT UN EXTRAIT DE LAMINARIA SACCHARINA

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**09.05.2012 Bulletin 2012/19**

(73) Proprietor: **The Procter & Gamble Company**
**Cincinnati, OH 45202 (US)**

(72) Inventors:
- **SWANSON, Cheri, Lynn**
  **West Chester, Ohio 45069 (US)**
- **HAKOZAKI, Tomohiro**
  **Cincinnati, Ohio 45252 (US)**
- **LAUGHLIN, Leo Timothy II**
  **Mason, Ohio 45040 (US)**

(74) Representative: **Sauvaître, Thibault Bruno**
**Procter & Gamble Service GmbH**
**Patent Department**
**PO Box 14**
**Frankfurter Straße 145**
**61476 Kronberg im Taunus (DE)**

(56) References cited:
**EP-A2- 1 997 537**
**WO-A1-98/26755**
**WO-A2-2009/047443**
**WO-A2-2010/088225**
**FR-A1- 2 838 340**

- **DATABASE GNPD [Online] Mintel; April 2008 (2008-04), "Extra Concentrated Brightening Essence", XP002610976, Database accession no. 887317**
- **"Laminaria saccharina extract" In: "International Cosmetic Ingredient Dictionary and Handbook, 11th Edition", 2006, The Cosmetics, Toiltery & Fragrance Association, Washington, USA, XP002610983, ISBN: 1-882626-34-4 the whole document**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to methods for improving the appearance of hyperpigmented spots in mammilian skin using a fast-acting and slow-acting agent.

BACKGROUND OF THE INVENTION

Human skin comprises three principal layers: the epidermis, the dermis, and the subcutaneous fat layer. The epidermis comprises four layers (from top to bottom): the stratum corneum, the granular layer, the spiny layer, and the basal layer. A separate fifth layer, the stratum lucidum, may be present between the stratum corneum and granular layer. The basal layer produces cells which gradually migrate upward to form the other epidermal layers. As these cells migrate upward, they lose their central nucleus and start to produce skin proteins (keratins) and fats (lipids). These cells are identified as keratinocytes when present in the upper layers of the epidermis. Melanocytes are another class of cells located in the basal layer of the epidermis. Melanocytes are responsible for the production of melanin, which is primary factor in skin pigmentation.

Melanin is produced by a complex set of reactions within the melanocyte involving, at a basic level, the enzyme tyrosinase and L-tyrosine as a substrate. Tyrosinase catalyzes the conversion of L-tyrosine to DOPA (L-3,4-dihydroxyphenylalanine) and of DOPA to dopaquinone. Dopaquinone undergoes further conversion to form melanin. Melanin aggregates in organelles known as the melanosomes which are transferred to keratinocytes along slender filaments of the melanocyte known as dendrites. There are approximately 1500 gene products expressed in melanosomes with 600 of them being expressed at any given time and 100 of them believed to be unique to the melanosome. In addition, there are many regulatory elements involved in signaling, in the transport of melanosomes within the melanocyte, and in the transfer of melanosomes to the keratinocytes.

The production of melanin can be triggered by a variety of external and internal events. For example, melanocytes produce additional melanin when skin is subjected to UV radiation. The melanin is then transported via melanasomes to the keratinocytes, which then leaves the skin with a "tanned" appearance. Once the UV light is removed the melanocytes return to normal levels of melanin production. Inflammation may initiate hyperpigmentation by direct stimulation of the melanocytes by mediators such as IL-1, endothelin-1, and/or stem cell factor. Reactive oxygen species, such as superoxide and nitric oxide, generated in damaged skin or released as by-products from inflammatory cells may be stimulators of melanocytes.

Over time, chronic UV exposure and other intrinsic and extrinsic aging factors may lead to permanent gene expression changes in keratinocytes and/or melanocytes resulting in age-related hyperpigmented spots. The mRNA levels of some melanogenesis associated genes (for example, tyrosinase, TYRP1) are reported to be increase actinic lentigos (age spots). There may also be accentuation of the epidermal endothelin cascade and a role for stem cell factor in hyperpigmentation. These changes can result in overproduction of melanin and resultant hyperpgimented spots that persist even when an insult, such as UV exposure, is avoided. Even beyond hyperpigmented spots, chronic UV exposure and other intrinsic and extrinsic aging factors may lead to more subtle changes in skin tone. Often these changes are described as uneven tone or as a mottled appearance. At least one study suggests that age spots can sometimes add 10 to 12 years of perceived age to a person and that melanin distribution can drive tone dependent age perception.

There is also a need to develop compositions for improving the appearance hyperpigmented spots that provide both rapid improvement and longer term improvement. Users want a composition that yields quick, noticeable improvement of hyperpigmented spots while also providing sustained, long-lasting improvement. Thus, there is a continuing desire to provide compositions and methods of treatment that can quickly and sustainably improve the appearance of hyperpigmented spots.

Extracts of *Laminaria Saccharina,* a species of brown algae, are known in the art. One example is sold under the tradename Phlorogine by Biotech Marine, France. Phlorogine is known as anti-seborrhoeic agent that can regulate the activity of sebaceous glands, as described for example in United States Patent Application Publication No. 2008/0119527A1. Extraction methods for brown algae are also known. European Patent No. 1074262B1 describes an extraction method for the class *Phaeophyceae* and the species *Laminaria Ochroleuca.* These extracts are described as being used in cosmetic compositions as an osmoprotector, free-radical scavenger, or against the effects of skin aging effects. A cosmetic composition sold under the brand name SK-II Facial Clear Solution (Procter & Gamble, Cincinnati, OH) has a concentration of Phlorogine of about 1.25%. The SK-II Facial Clear Solution is marketed as a gel hydrator that moisturizes the skin without increasing oily shine.

## SUMMARY OF THE INVENTION

A method of improving the appearance of a hyperpigmented spot comprising:

a. inspecting the facial skin surface for hyperpigmented spots in need of treatment based on size and/or color;
b. identifying a hyperpigmented spot on the facial skin surface in need of treatment; and
c. applying a composition to the hyperpigmented spot on a facial skin surface, the composition comprising:

i. a safe and effective amount of an fast-acting agent for hyperpigmented spots, wherein the fast-acting agent comprises *Laminaria Saccharina* extract, and
ii. a safe and effective amount of a slow-acting agent for hyperpigmented spots, wherein the slow-acting agent comprises vitamin B3;

wherein the composition is applied at least daily for a period of time sufficient for both the fast-acting agent and slow-acting agent to improve the appearance of the hyperpigmented spot.

In response to the technical problems identified in the background, the present invention may take other forms. As will be appreciated more fully in the description, the inventors have surprisingly found that fast-acting actives for treating hyperpigmented spots do not necessarily provide the best sustained improvement. To address this problem, compositions and methods are described herein utilizing a fast-acting active and a slow-acting active for improvement of hyperpigmented spots. Further forms of the present invention will be appreciated in the detailed description that follows.

## BRIEF DESCRIPTION OF THE DRAWINGS

It is believed that the present invention will be better understood from the following description taken in conjunction with the accompanying drawings. The referenced drawings are not to be construed as limiting the scope of present invention.

Figure 1 is an exemplary applicator in the form of a dropper.
Figure 2 is an exemplary applicator in the form of a wand.
Figure 3 is an exemplary applicator in the form of a narrow-tip tube.
Figure 4 is a full color image of a participant.
Figure 5 is a melanin concentration map of the same participant as shown in Figure 4.

## DETAILED DESCRIPTION OF THE INVENTION

All percentages and ratios used herein are by weight of the total composition and all measurements made are at 25°C, unless otherwise designated. All numeric ranges are inclusive of narrower ranges; delineated upper and lower range limits are interchangeable to create further ranges not explicitly delineated.

The compositions of the present invention can comprise, consist essentially of, or consist of, the essential components as well as optional ingredients described herein. As used herein, "consisting essentially of" means that the composition or component may include additional ingredients, but only if the additional ingredients do not materially alter the basic and novel characteristics of the claimed compositions or methods.

The term "apply" or "application", as used in reference to a composition, means to apply or spread the compositions of the present invention onto a human skin surface such as the epidermis.

The term "dermatologically acceptable," as used herein, means that the compositions or components described are suitable for use in contact with human skin tissue without undue toxicity, incompatibility, instability, allergic response, and the like.

The term "safe and effective amount" as used herein means an amount of a compound or composition sufficient to significantly induce a positive benefit.

The term "post-inflammatory hyperpigmentation" as used herein refers to an acute to chronic increase in pigmentation as a response to a transient inflammatory event. Post-inflammatory hyperpigmentation is particularly prevalent in, but not limited to, dark skin subjects. Post-inflammatory hyperpigmentation typically subsides once the transient inflammatory event dissipates. Examples of transient inflammatory events include, but are not limited to, acne lesions, ingrown hairs, scratches, insect bites, surfactant damage, and short-term UV exposure.

The term "hyperpigmented spot" as used herein refers to a defined area of skin wherein the pigmentation is greater than that of an adjacent area of skin due to localized and chronic or systemic overproduction of melanin. Hyperpigmented spots typically are between about 2 mm and about 10 mm in diameter but smaller or larger spots are possible. Hyperpigmented spots can include one or more of age spots, sun spots, solar lentigos, hypo-melanotic lesions, freckles,

and melasma spots.

The term "age spots" as used herein refers to a defined area of skin wherein the pigmentation is greater than that of adjacent skin due to localized and chronic overproduction of melanin caused by intrinsic or extrinsic aging factors.

The term "skin tone" as used herein refers to the overall appearance of melanin in the skin caused by the systemic, rather than transient, synthesis of melanin. Skin tone is typically characterized over a larger area of the skin. The area ideally may be than 100 $mm^2$, but larger areas are envisioned such as the entirety of the facial skin or any of the facial skin surfaces. Skin tone can be measured by image analysis. For example, overall lightness can be measured by L* coordinate in L*a*b* color space (International Commission on Illumination). Chromophore mapping such as melanin mapping and melanin concentration may be used as an indicator of overall skin tone. Mean melanin may be calculated from the chromophore map data. Additionally, skin tone evenness can be determined by melanin evenness which also may be calculated from the chromophore map data. Suitable chromophore mapping techniques are discussed in the example below.

The term "facial skin surfaces" as used herein refers to one or more of forehead, periorbital, cheek, perioral, chin, and nose skin surfaces.

The term "fast-acting" as used herein means providing a noticeable improvement (e.g., size or darkness) in hyperpigmented spots in less than or equal to about 4 weeks.

The term "slow-acting" as used herein means providing a noticeable improvement (e.g., size or darkness) in hyperpigmented spots after about 4 weeks to about 8 weeks.

I. Compositions

The present invention relates to a method in which various compositions are used more specifically, to compositions for topical application to a skin surface. The compositions may be made into a wide variety of product forms that include, but are not limited to, solutions, suspensions, lotions, creams, gels, toners, sticks, pencil, sprays, aerosols, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks (with and without insoluble sheet), make-up such as foundations, eye liners, and eye shadows, and the like. The composition form may follow from the particular dermatologically acceptable carrier chosen, if present in the composition.

A. Fast-Acting Agent for Hyperpigmented Spots ("Fast-Acting Agent")

Compositions used in the present invention include a fast-acting agent for the improvement of a hyperpigmented spot. In certain embodiments, the fast-acting agent may include a safe and effective amount of *Laminaria Saccharina* extract, a brown algae extract. A preferred extract is Phlorogine and/or Phlorogine BG, which is available from Marine Biotech, France. Another suitable *Laminaria Saccharina* extract is available via product code HG 657 from Ennagram, France. The composition may contain Phlorogine and/or Phlorogine BG in an amount from 0.008% to 50%, in one embodiment from about 0.04% to 20%, in another embodiment from 0.2% to 10%, by weight of the composition. In yet another embodiment the composition comprises from 1% to 5%, and in yet another embodiment from 1% to 3% Phlorogine and/or Phlorogine BG by weight of the total composition.

The *Laminaria Saccharina* extract may include other compounds, such as, for example water, thickeners, humectants, solvents and solubilizers, etc. For example, Phlorogine and/or Phlorogine BG contain approximately about 1% to about 2.5% dry extract with the remaining material being inert carrier. The composition of the present invention therefore may contain a *Laminaria Saccharina* extract in an amount from about 0.00008% to about 1.25%, in one embodiment from about 0.0004% to about 0.5%, in another embodiment from about 0.002% to about 0.25%, by weight of the composition. In yet another embodiment the composition comprises from about 0.01% to about 0.125%, and in yet another embodiment from 0.01% to *0.075% Laminaria Saccharina* extract by weight of the total composition. The *Laminaria Saccharina* extract can be prepared by processes known in the art, such as, for example, described in European Patent No. 1074262B1.

In certain embodiments, the fast-acting agent may include a safe and effective amount of N-acyl Amino Acid Compound. The amino acid can be one of any of the amino acids known in the art. N-acyl amino acid compound includes N-acyl amino acids, their isomers, their salts, and derivatives thereof. The composition of the present invention may comprise from about 0.0001 to about 25%, from about 0.001 to about 10%, from about 0.01 to about 5%, or from about 0.02 to about 2.5%, by weight of the composition, of the N-acyl amino acid. The N-acyl amino acid compounds of the present invention correspond to Formula I:

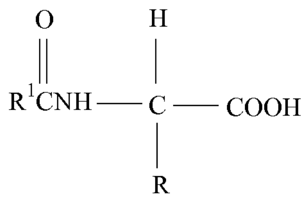

Formula I

wherein R can be a hydrogen, alkyl (substituted or unsubstituted, branched or straight chain), aryl, or a combination of alkyl and aromatic groups. A list of possible side chains of amino acids known in the art are described in Stryer, Biochemistry, 1981, published by W.H. Freeman and Company. $R^1$ can be $C_1$ to $C_{30}$, saturated or unsaturated, straight or branched, substituted or unsubstituted alkyls; substituted or unsubstituted aromatic groups; or mixtures thereof.

The N-acyl amino acid compound may be selected from the group consisting of N-acyl phenylalanine, N-acyl tyrosine, their isomers, their salts, and derivatives thereof. The amino acid can be the D or L isomer or a mixture thereof. N-acyl phenylalanine corresponds to the following Formula II:

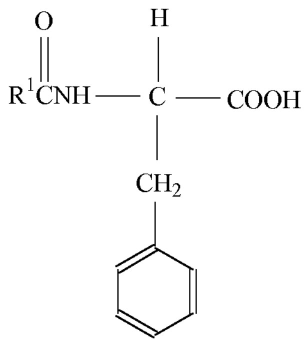

Formula II

wherein $R^1$ can be $C_1$ to $C_{30}$, saturated or unsaturated, straight or branched, substituted or unsubstituted alkyls; substituted or unsubstituted aromatic groups; or mixtures thereof.

N-acyl tyrosine corresponds to the following Formula III:

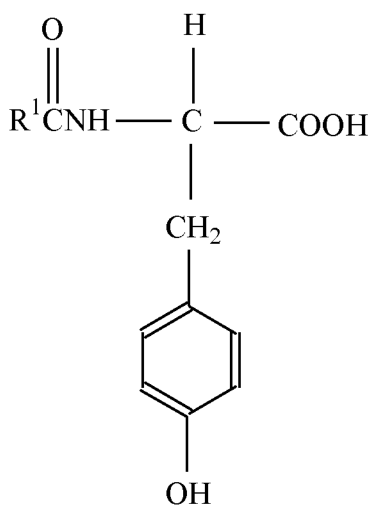

Formula III

wherein $R^1$ can be $C_1$ to $C_{30}$, saturated or unsaturated, straight or branched, substituted or unsubstituted alkyls; substituted or unsubstituted aromatic groups; or mixtures thereof.

In one embodiment, the N-acyl Amino Acid Compound comprises N-undecylenoyl-L-phenylalanine. This agent belongs to the broad class of N-acyl phenylalanine derivatives, with its acyl group being a C11 mono-unsaturated fatty acid moiety and the amino acid being the L-isomer of phenylalanine. N-undecylenoyl-L-phenylalanine corresponds to

the following Formula IV:

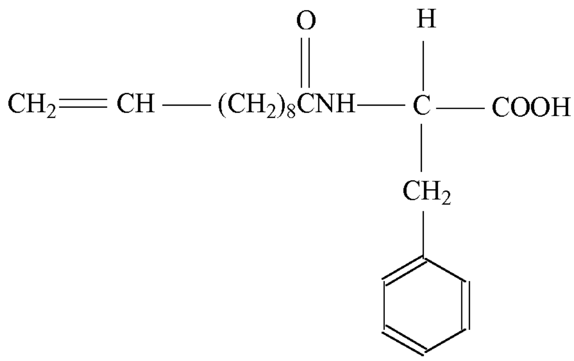

Formula IV

N-undecylenoyl-L-phenylalanine is commercially available under the tradename Sepiwhite® from SEPPIC.

In a certain embodiments, the fast-acting agent may comprise a safe and effective amount of N-acyl Amino Acid Compound and a safe and effective amount of *Laminaria Saccharina* extract

B. Slow-Acting Agent for Hyperpigmented Spots ("Slow-Acting Agent")

Compositions used in the present invention include a slow-acting agent for the improvement of a hyperpigmented spot. When present, the compositions of the present invention contain up to about 50%, 40%, 30%, 20%, 10%, 5%, or 3%, by weight of the composition, of the slow-acting agent. When present, the compositions of the present invention contain at least about 0.001%, 0.01%, 0.1%, 0.2%, 0.5%, or 1%, by weight of the composition, of the slow-acting agent. Suitable ranges include any combination of the lower and upper limits including suitable ranges from about 0.1% to about 50%; from about 0.2% to about 20%; or from about 1% to about 10%, by weight of the composition, of the additional slow-acting agent. The amounts listed herein are only to be used as a guide, as the optimum amount of the slow-acting agent will depend on the specific active selected.

Suitable slow-acting agent include, but are not limited to, sugar amines, vitamin B3 compounds, 1,3-dihydroxy-4-alkylbenzene such as hexylresorcinol, arbutin, deoxyarbutin, sucrose dilaurante, bakuchoil (4-[(1E, 3S)-3-ethenyl-3,7-dimethyl - 1,6 octadienyl] phenol or monterpene phenol), pyrenoine (available from Biotech Marine, France), panicum miliaceum seed extract, arlatone dioic acid, cinnamic acid, ferulic acid, achromaxyl, methyl nicotinamide, oil soluble licorice extract, folic acid, undecylenic acid (i.e., undecenoic acid), zinc undecylenate, thiamine (Vitamin B1) and its hydrochloride, L-tryptophan, hexylrescorcinol, helianthus annuus (sunflower) and vitis vinifera (grape) leaf extract, carnosine (i.e., dragosine), methyl gentisate, 1,2-hexandiol and 1,2-octandiol (i.e., combination sold as Symdiol 68 by Symrise AG, Germany), inositol, N-acyl Amino Acid compounds such as N-undecylenoyl-L-phenylalanine, koijic acid, hexamidine compounds, salicylic acid, and retinoids including retinol and retinyl propionate.

In certain embodiments, the additional slow-acting agent comprises vitamin B3 compounds. In certain embodiments, the additional slow-acting agent comprises niacinamide.

As used herein, "vitamin $B_3$ compound" means a compound having the formula:

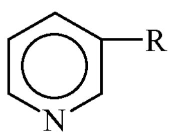

wherein R is - $CONH_2$ (*i.e.*, niacinamide), - COOH (*i.e.*, nicotinic acid) or - $CH_2OH$ (*i.e.*, nicotinyl alcohol); derivatives thereof; and salts of any of the foregoing. As used herein, "sugar amine" includes isomers and tautomers of such and its salts (*e.g.,* HCl salt) and its derivatives. Examples of sugar amines include glucosamine, N-acetyl glucosamine, mannosamine, N-acetyl mannosamine, galactosamine, N-acetyl galactosamine, their isomers (*e.g.*, stereoisomers), and their salts (*e.g.*, HCl salt). As used herein, "hexaminide compound" means a compound having the formula:

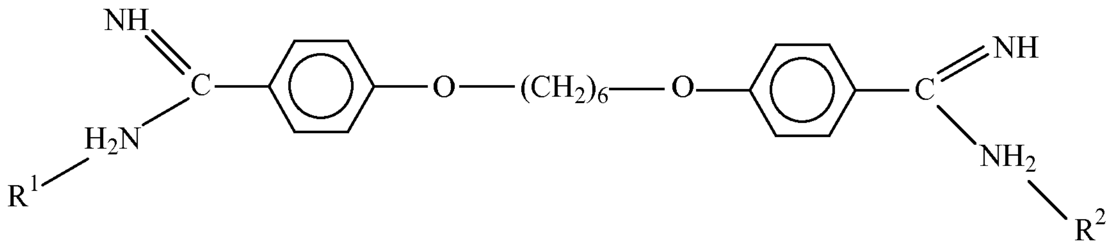

wherein $R^1$ and $R^2$ are optional or are organic acids (*e.g.*, sulfonic acids, etc.). In one embodiment, hexamidine compound includes hexamidine diisethionate.

C. Anti-Inflammatory Agents

Hyperpigmentation may result from skin inflammation. Transient inflammatory events triggering hyperpigmentation and, more specifically, post-inflammatory hyperpigmentation include, but are not limited to, acne lesions, ingrown hairs, scratches, insect bites, surfactant damage, and short-term UV exposure. Inflammation induced hyperpigmentation including post-inflammatory hyperpigmentation may be managed by incorporating into the compositions of the present invention an anti-inflammatory agent. When present, the compositions of the present invention contain up to about 20%. 10%. 5%, 3%, or 1% by weight of the composition, of the anti-inflammatory agent. When present, the compositions of the present invention contain at least about 0.001%, 0.01%, 0.1%, 0.2%, 0.3% 0.5%, or 1%, by weight of the composition, of the anti-inflammatory agent. Suitable ranges include any combination of the lower and upper limits. Suitable anti-inflammatory agents include, but are not limited to nonsteroidal anti-inflammatory agents (NSAIDS including but not limited to ibuprofen, naproxen, flufenamic acid, etofenamate, aspirin, mefenamic acid, meclofenamic acid, piroxicam and felbinac), glycyrrhizic acid (also known as glycyrrhizin, glycyrrhixinic acid, and glycyrrhetinic acid glycoside) and salts such as dipotassium glycyrrhizate, glycyrrhetenic acid, licorice extracts, candelilla wax, bisabolol (e.g., alpha bisabolol), manjistha (extracted from plants in the genus *Rubia,* particularly *Rubia cordifolia*), and guggal (extracted from plants in the genus *Commiphora,* particularly *Commiphora mukul*), kola extract, chamomile, red clover extract, and sea whip extract, derivatives of any of the foregoing, and mixtures thereof.

D. Sunscreen Actives

The compositions may comprise one or more sunscreen actives (or sunscreen agents) and/or ultraviolet light absorbers. Herein, "sunscreen active" includes both sunscreen agents and physical sunblocks. Sunscreen actives and ultraviolet light absorbers may be organic or inorganic. Examples of suitable sunscreen actives and ultraviolet light absorbers are disclosed in Personal Care Product Council's International Cosmetic Ingredient Dictionary and Handbook, Thirteenth Edition, as "sunscreen agents." Particularly suitable sunscreen actives are 2-ethylhexyl-p-methoxycinnamate (commercially available as PARSOL™ MCX), 4,4'-t-butyl methoxydibenzoyl-methane (commercially available as PARSOL™ 1789), 2-hydroxy-4-methoxybenzophenone, octyldimethyl-p-aminobenzoic acid, digalloyltrioleate, 2,2-dihydroxy-4-methoxybenzophenone, ethyl-4-(bis(hydroxypropyl))aminobenzoate, 2-ethylhexyl-2-cyano-3,3-diphenylacrylate, 2-ethylhexyl-salicylate, glyceryl-p-aminobenzoate, 3,3,5-trimethylcyclohexylsalicylate, menthyl anthranilate, p-dimethyl-aminobenzoic acid or aminobenzoate, 2-ethylhexyl-p-dimethyl-amino-benzoate, 2-phenylbenzimidazole-5-sulfonic acid, 2-(p-dimethylaminophenyl)-5-sulfonicbenzoxazoic acid, octocrylene, zinc oxide, benzylidene camphor and derivatives thereof, titanium dioxide, and mixtures thereof.

In one embodiment, the composition may comprise from about 1% to about 20%, and alternatively from about 2% to about 10% by weight of the composition, of the sunscreen active and/or ultraviolet light absorber. Exact amounts will vary depending upon the chosen sunscreen active and/or ultraviolet light absorber and the desired Sun Protection Factor (SPF), and are within the knowledge and judgment of one of skill in the art.

E. Optional Components

The compositions of the present invention may contain a variety of other ingredients that are conventionally used in given product types provided that they do not unacceptably alter the benefits of the invention. When present, compositions of the present invention may contain from about 0.0001% to about 50%; from about 0.001% to about 20%; or, alternately, from about 0.01% to about 10%, by weight of the composition, of the optional components. The amounts listed herein are only to be used as a guide, as the optimum amount of the optional components used in a composition will depend on the specific active selected since their potency does vary considerably. Hence, the amount of some optional components useful in the present invention may be outside the ranges listed herein.

The optional components, when incorporated into the composition, should be suitable for use in contact with human skin tissue without undue toxicity, incompatibility, instability, allergic response, and the like within the scope of sound judgment. The compositions of the present invention may include optional components such as anti-acne actives, desquamation actives, anti-cellulite agents, chelating agents, flavonoids, tanning active, non-vitamin antioxidants and radical scavengers, hair growth regulators, anti-wrinkle actives, anti-atrophy actives, minerals, phytosterols and/or plant hormones, N-acyl amino acid compounds, antimicrobial or antifungal actives, and other useful skin care actives, which are described in further detail in U.S. application publication No. US2006/0275237A1 and US2004/0175347A1.

The Personal Care Product Council's International Cosmetic Ingredient Dictionary and Handbook, Thirteenth Edition, describes a wide variety of non-limiting cosmetic and pharmaceutical ingredients commonly used in the skin care industry, which are suitable optional components for use in the compositions of the present invention. Examples of these ingredient classes include: abrasives, absorbents, aesthetic components such as fragrances, pigments, colorings/colorants, essential oils, anti-caking agents, antifoaming agents, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, and thickeners.

F. Dermatologically Acceptable Carrier

The compositions of the present invention may also comprise a dermatologically acceptable carrier ("carrier") for the composition. The phrase "dermatologically acceptable carrier", as used herein, means that the carrier is suitable for topical application to the keratinous tissue, has good aesthetic properties, is compatible with the actives of the present and will not cause any safety or toxicity concerns. In one embodiment, the carrier is present at a level of from about 50% to about 99%, about 60% to about 98%, about 70% to about 98%, or, alternatively, from about 80% to about 95%, by weight of the composition.

The carrier can be in a wide variety of forms. Non-limiting examples include simple solutions (aqueous or oil based), emulsions, and solid forms (gels, sticks, flowable solids, amorphous materials). In certain embodiments, the dermatologically acceptable carrier is in the form of an emulsion. Emulsion may be generally classified as having a continuous aqueous phase (*e.g.*, oil-in-water and water-in-oil-in-water) or a continuous oil phase (*e.g.*, water-in-oil and oil-in-water-in-oil). The oil phase of the present invention may comprise silicone oils, non-silicone oils such as hydrocarbon oils, esters, ethers, and the like, and mixtures thereof.

The aqueous phase typically comprises water. However, in other embodiments, the aqueous phase may comprise components other than water (non-water components), including but not limited to water-soluble moisturizing agents, conditioning agents, anti-microbials, humectants and/or other water-soluble skin care actives. In one embodiment, the non-water component of the composition comprises a humectant such as glycerin and/or other polyols. However, it should be recognized that the composition may be substantially (*i.e.*, less than 1% water) or fully anhydrous.

A suitable carrier is selected to yield a desired product form. Furthermore, the solubiluity or dispersibility of the compositions components (*e.g.*, *Laminaria Saccharina* extract, sunscreen active, additional components) may dictate the form and composition of the carrier. In one embodiment, oil-in-water or water-in-oil emulsions are preferred.

Emulsions may further comprise an emulsifier. The composition may comprise any suitable percentage of emulsifier to sufficiently emulsify the carrier. Suitable weight ranges include from about 0.1% to about 10% or about 0.2% to about 5% of an emulsifier, based on the weight of the composition. Emulsifiers may be nonionic, anionic or cationic. Suitable emulsifiers are disclosed in, for example, U.S. Patent 3,755,560, U.S. Patent 4,421,769, and McCutcheon's Detergents and Emulsifiers, North American Edition, pages 317-324 (1986). Suitable emulsions may have a wide range of viscosities, depending on the desired product form.

The carrier may further comprise a thickening agent as are well known in the art to provide compositions having a suitable viscosity and rheological character.

II. Exemplary Compositions

In the examples, all concentrations are listed as weight percent, unless otherwise specified and may exclude minor materials such as diluents, filler, and so forth. The listed formulations, therefore, comprise the listed components and any minor materials associated with such components. As is apparent to one of ordinary skill in the art, the selection of these minor materials will vary depending on the physical and chemical characteristics of the particular ingredients selected to make the present invention as described herein.

The Examples may be used for general or localized application and treatment for one or more hyperpigmented spots. Ex. B and Ex. G are comparative examples

| Component | Ex. A | Ex. B | Ex. C | Ex. D | Ex. E | Ex. F | Ex. G |
|---|---|---|---|---|---|---|---|
| Phlorogine or Phlorogine BG*1 | 1.000 | 1.000 | 2.000 | 1.000 | 1.000 | 1.000 | 1.000 |
| N-Acetylglucosamine | 0 | 0 | 0 | 0 | 0 | 2.000 | 0 |
| Hexamidine Diisethionate | 0 | 0 | 0 | 0.090 | 0 | 0 | 0 |
| Undecylenoyl-phenylalanine*2 (neutralized) | 0 | 0 | 0 | 0 | 1.000 | 0.500 | 1.000 |
| Dipotassium Glycyrrhizate | 0 | 0 | 0 | 0.100 | 0.300 | 0.100 | 0 |
| Niacinamide | 5.000 | 0 | 5.000 | 5.000 | 5.000 | 5.000 | 0 |
| Hexylresorcinol | 0 | 0.400 | 0 | 0 | 0 | 0 | 0 |
| Isohexadecane | 3.000 | 3.000 | 3.000 | 3.000 | 3.000 | 3.000 | 3.000 |
| Isopropyl isostearate | 1.330 | 1.330 | 1.330 | 1.330 | 1.330 | 1.330 | 1.330 |
| Cetearyl glucoside + cetearyl alcohol *3 | 0.200 | 0.200 | 0.200 | 0.200 | 0.200 | 0.200 | 0.200 |
| Behenyl alcohol | 0.400 | 0.400 | 0.400 | 0.400 | 0.400 | 0.400 | 0.400 |
| Cetyl alcohol | 0.320 | 0.320 | 0.320 | 0.320 | 0.320 | 0.320 | 0.320 |
| Stearyl alcohol | 0.480 | 0.480 | 0.480 | 0.480 | 0.480 | 0.480 | 0.480 |
| Tocopheryl acetate | 0.500 | 0.500 | 0.500 | 0.500 | 0.500 | 0.500 | 0.500 |
| PEG-100 stearate | 0.100 | 0.100 | 0.100 | 0.100 | 0.100 | 0.100 | 0.100 |
| Glycerin | 7.000 | 7.000 | 7.000 | 7.000 | 7.000 | 7.000 | 7.000 |
| Polyacrylamide + C13-14 isoparaffin + laureth-7 *4 | 2.000 | 2.000 | 2.000 | 2.000 | 2.000 | 2.000 | 2.000 |
| Disodium EDTA | 0.100 | 0.100 | 0.100 | 0.100 | 0.100 | 0.100 | 0.100 |
| Benzyl alcohol | 0.400 | 0.400 | 0.400 | 0.400 | 0.400 | 0.400 | 0.400 |
| Dimethicone + dimethiconol *5 | 2.000 | 2.000 | 2.000 | 2.000 | 2.000 | 2.000 | 2.000 |
| Homosalate | 0 | 0 | 0 | 9.000 | 0 | 0 | 0 |
| Avobenzone | 0 | 0 | 0 | 3.000 | 0 | 0 | 0 |
| Octocrylene | 0 | 0 | 0 | 2.600 | 0 | 0 | 0 |
| Oxybenzone | 0 | 0 | 0 | 1.000 | 0 | 0 | 0 |
| Octisalate | 0 | 0 | 0 | 4.500 | 0 | 0 | 0 |
| Water | QS | QS | QS | QS | QS | QS | QS |
| TOTAL | 100 | 100 | 100 | 100 | 100 | 100 | 100 |

*1 - Available from Biotech Marine, France.
*2 - Sepiwhite available from SEPPIC, France.
*3 - Emulgade PL 68/50 available from Cognis GmbH.
*4 - Sepigel 305, available from SEPPIC, France.
*5 - Dow Corning DC1503 available from Dow Corning. Inc Midland. MT.

The compositions are generally prepared by conventional methods such as are known in the art of making topical compositions. Such methods typically involve mixing of the ingredients in one or more steps to a relatively uniform state, with or without heating, cooling, application of vacuum, and the like. Typically, emulsions are prepared by first mixing the aqueous phase materials separately from the fatty phase materials and then combining the two phases as appropriate to yield the desired continuous phase. The compositions are preferably prepared such as to optimize stability (physical stability, chemical stability, photostability) and/or delivery of the active materials. This optimization may include appropriate pH (e.g., less than 7), exclusion of materials that can complex with the active agent and thus negatively impact stability or delivery (e.g., exclusion of contaminating iron), use of approaches to prevent complex formation (e.g., appropriate dispersing agents or dual compartment packaging), use of appropriate photostability approaches (e.g.,

incorporation of sunscreen/sunblock, use of opaque packaging), etc.

III. Methods of Treatment

Various methods of treatment, application, regulation, or improvement may utilize the aforementioned compositions. The method of the present invention includes the step of identifying a hyperpigmented spot for improvement by the composition. The hyperpigmented spot may be identified by the user or a third party such as a dermatologist, cosmetician, or other caregiver. Identification is done by visual inspection of the skin for hyperpigmented spots in need of treatment based on size and/or color. Identification may also be done by commercially available imaging devices such SIAscope V (available from Astron Clinica, Ltd., UK) or the VISIA® Complexion Analysis system (available from Canfield Scientific, Inc., Fairfield, NJ). Both devices are capable of collecting images of the skin and identifying hyperpigmented spots. In some instances, the method comprises the step of identifying a plurality of hyperpigmented spots for treatment by the composition. All of the following steps are applicable to both a single hyperpigmented spot as well as a plurality of hyperpigmented spots.

Identification of the hyperpigmented spot may occur on any skin surface of the body. Skin surfaces of the most concern tend to be those not typically covered by clothing such as facial skin surfaces, hand and arm skin surfaces, foot and leg skin surfaces, and neck and chest skin surfaces (e.g., décolletage). In particular, identification of the hyperpigmented spot of the facial skin surfaces include the forehead, perioral, chin, periorbital, nose, and/or cheek skin surfaces.

The method comprises the step of applying the composition to the hyperpigmented spot or spots on the facial skin surface. Many regimens exist for the application of the composition to the hyperpigmented spot. The composition may be applied at least once a day, and more preferably twice a day, during a treatment period. When applied twice daily, the first and second applications are separated by at least 1 to about 12 hours. Typically, the composition may be applied in the morning and/or in the evening typically before bed.

The treatment period is ideally of sufficient time to provide an improvement in the hyperpigmented spot. The improvement may be a detectable reduction in size of the hyperpigmented spot, lightening of the hyperpigmented spot (*e.g.*, lighter in color), or decrease in melanin of the hyperpigmented spot. The treatment period may be at least about 1 week. The treatment period may last about 4 weeks or about 8 weeks. In certain embodiments, the treatment period will extend over multiple months (*i.e.*, 3-12 months) or multiple years. In one embodiment the composition is applied to the hyperpigmented spot(s) at least once a day during a treatment period of at least about 4 weeks or at least about about 8 weeks. In one embodiment the composition is applied to the hyperpigmented spot(s) twice a day during a treatment period of at least about 4 weeks or 8 weeks.

The step of applying the composition to the hyperpigmented spot may be done by localized application. In reference to application of the composition, the term "localized", "local", or "locally" mean that the composition is delivered the targeted area (such as the hyperpigmented spot) while minimizing delivery to skin surface not requiring treatment. The composition may be applied and lightly massaged into the hyperpigmented spot. It is recognized that localized application does allow for a reasonable amount of the composition to be applied to areas adjacent the hyperpigmented spot (*i.e.*, the composition is unlikely to be applied or to remain within the boundary of the hyperpigmented spot without some spreading). The form of the composition or the dermatologically acceptable carrier should be selected to facilitate localized application. While certain embodiments of the present invention contemplate applying a composition locally to a hyperpigmented spot, it will be appreciated that compositions of the present invention can be applied more generally or broadly to one or more facial skin surfaces to reduce the appearance of hyperpigmented spots within those facial skin regions.

In some embodiments, the composition may be delivered by a variety of applicators appropriate for localized and general application. Several applicators are shown, by way of example, in Figures 1-3. In Figure 1, a suitable applicator 10 may be a dropper 12 which is shown with a bottle 14 that may contain the composition. Figure 2 shows an applicator 20 as a wand 22 with a housing 24 that may contain the composition. The wand 22 may comprise a handle 26, a stem 27, and an applicator head 28. The applicator head 28 may comprise fibers, foam, cotton, or any other suitable material that may releasably hold the composition. Figure 3 shows an applicator 30 as a narrow-tip tube 32 with a body 34 and narrow dispensing tip 36. The composition may be stored within the body 34 and dispensed through the pointed tip 36. Other applicators that can apply first composition locally to the hyperpigmented spot may also be used such as a cotton swab. Other suitable applicators include SH-0127 pen applicator available from Shya Hsin Plastic Works, Inc., Taiwan and either the Xpress Tip or liquid filled swab available from SwabPlus, Inc., China. The applicator may be configured to easily apply the composition to hyperpigmented spots having an approximate diameter between about 2 mm and about 10 mm and allowing for a dosed amount of the composition of between about 1 to about 50 $uL/cm^2$ or between about 1 to about $5uL/cm^2$. the composition is applied to the one or more hyperpigmented spots and more generally to one or more facial skin surfaces contemporaneously (*i.e.*, over a period of less than 30 minutes or, more typically, less than 5 minutes).

While some methods described herein contemplate applying the compositions of the present invention with an

applicator, it will be appreciated that applicators are not required and the compositions of the present invention can also be applied directly or using one's finger or in other conventional manners.

Suitable methods may comprise any one or more of the abovementioned steps. All of the aforementioned steps are applicable to application, treatment, regulation, and/or improvement of both a single hyperpigmented spot as well as a plurality of hyperpigmented spots. Likewise, the exemplary methods that follow are applicable to both a single hyperpigmented spot as well as a plurality of hyperpigmented spots.

The method of the present invention of improving the appearance of a hyperpigmented spot comprises the step of applying a composition comprising a fast-acting agent and a slow-acting agent to a hyperpigmented spot on a facial skin surface, wherein the composition is applied at least daily for a period of time sufficient for both the fast-acting agent and slow-acting agent to improve the appearance of the hyperpigmented spot. The method of improving the appearance of hyperpigmented spot of the present invention comprises the steps of identifying a hyperpigmented spot on a facial skin surface and of applying a composition comprising a fast-acting agent and a slow-acting agent to a hyperpigmented spot on a skin surface, wherein the composition is applied at least daily for a period of time sufficient for both the fast-acting agent and slow-acting agent to improve the appearance of the hyperpigmented spot.

IV. Test Methods

The following methods are provided to illustrate certain features and advantages of various embodiments of the invention and should not be construed as limiting the scope thereof.

A. Melanin Synthesis Assay

A B16-F1 mouse melanoma cell line is employed in the assay. The B16-F1 cells are obtained from American Tissue Culture Collection, Virginia, USA. The cell culture medium used in the assay comprises 500 mL of Dulbecco's Modified Eagle's Medium (DMEM), 50 mL Fetal Bovine Serum (FBS), and 5 mL of penicillin-streptomycin liquid. B16-F1 cells that are cultured in this medium and grown to greater than 90% confluency synthesize melanin. While not intending to be bound by any theory, it is hypothesized that the melanin synthesis is stimulated by the culture medium and/or stress induced by growth to a high confluency. The DMEM and FBS can be obtained from American Tissue Culture Collection and the penicillin-streptomycin liquid can be obtained from Invitrogen, Inc., California, USA. Equipment used in the assay include a $CO_2$ incubator, such as a Forma Series Model 3110 by Therma Scientific, Massachusets, USA; a Hemocytometer, such as a Bright Line model by Hauser Scientific, Pennsylvania, USA; and a UV-Visible Spectrum Plate Reader, such as a SpectraMax250 from Molecular Devices, California, USA. The assay steps include:

1. Day 0 - Cell Growth: Warm the cell culture medium to 37°C and place 29 mL into a T-150 flask. Add approximately 1 x$10^6$ of B16-F1 passage 1 mouse cells to the T-150 flask and incubate for 3 days at 37°C, 5% $CO_2$, 90% relative humidity, until about 80% confluency.
2. Day 3 - Initiate a 96 Well Plate: At day 3, trypsinize the cells from the T-150 flask and determine the concentration of cells using the Hemacytometer. Initiate a 96 well plate with 2,500 cells per well in 100 uL of cell culture medium. Incubate the plate at 37°C, 5% $CO_2$, 90% relative humidity for 2 days until at least 20% to 40% confluent.
3. Day 5 - Remove the cell culture medium from the plate and replace with fresh culture medium (100uL per well). Add 1uL of Phlorogine diluted in water. Multiple dilution ratios may be tested in order to generate a dose response curve, wherein preferably three wells are treated with each dilution ratio. Controls comprise wells having the cell culture medium, B16-F1 cells, and the solvent (control #1); wells comprising the cell culture medium and the solvent (control #2); and optionally wells comprising the cell culture medium, solvent and [test compound] when necessary to control for the [test compound] background color (control #3).
4. Day 7 - Measure Melanin Production: Cells should have a confluency greater than about 80%. If not, this data point is not used. Add 100 uL of a 0.75% sodium hydroxide solution to each well. Read the 96 well plate using the UV-Vis Plate Reader at 410 nm to optically measure the amount of melanin produced between wells that are treated with [test compound] and control wells that are not. Wells in which melanin is produced appear brownish in color. Wells in which little melanin is produced appear clear to light purple in color. Percentage of melanin synthesis inhibition is calculated by the following equation:

$$\frac{100 - [OD_{410\ \text{Test Compound}} - OD_{410\ \text{Control \#2}}]}{(OD_{410\ \text{Control \#1}} - OD_{410\ \text{Control \#2}})} \times 100$$

Where $OD_{410}$ is the Optical Density at 410 nm as measured by the UV-Vis Spectrum Plate Reader.

When Control #3 is used, the formula for percentage melanin synthesis inhibition is:

$$\frac{100 - [OD_{410\ \text{Test Compound}} - OD_{410\ \text{Control \#3}}]}{(OD_{410\ \text{Control \#1}} - OD_{410\ \text{Control \#2}})} \times 100$$

Using generally the assay outlined above, melanin synthesis in Phlorogine treated B16-F1 cells was inhibited as compared to control cells as shown below in Table 1.

Table 1

| Phlorogine Concentration (w/v%) | 1% | 0.2% | 0.04% | 0.008% | 0.0016% | 0.000064% |
|---|---|---|---|---|---|---|
| approximate *Laminaria Saccharina* extract concentration | 0.025%-0.01% | 0.005%-0.002% | 0.001%-0.0004% | 0.0002%-0.00008% | 0.00004%-0.000016% | 0.0000016%-0.00000064% |
| Percentage melanin synthesis inhibition | 48.1% | 11.4% | 5.5% | 5.5% | -3% | -1.6% |
| Confluency (visual inspection) | >90% | >90% | >90% | >90% | >90% | >90% |

B. *In Vivo* Testing for Hyperpigmented Spot Reduction and Melanin Evenness

A 9 week *in vivo* study was conducted using a round robin, vehicle controlled, split face design including a 1 week normalization period with 270 subjects. The 270 subjects were screened according to inclusion/exclusion criteria which included the following:

Inclusion

1. Has hyperpigmented spots around cheek and/or periorbital area on both sides of the face.

2. Has at least 1 hyperpigmented spot of 8-10 mm diameter, 4 spots of 4-6 mm or 10 spots of 2-3 mm diameter (sun spots, freckles, or melasma spots) or equivalent spot area in the cheek area on each side of their face.

3. Is willing to refrain from sun exposure by using supplied UV lotion and physical UV blocks, such as a hat, to avoid facial sunburn, tanning or wind burn.

Exclusion

1. Has been diagnosed as having atopy, eczema, psoriasis, or other chronic skin diseases.

2. Has obvious signs of facial skin disease (e.g., more than 5 pimples, areas of red scaling skin, superficial thin blood vessels, etc.).

3. Has significant areas of discoloration or scarring on the face.

4. Has more than 3 prominent moles (> 3mm) on the face.

Two hundred and seventy subjects were recruited for the study. Approximately 60 subjects dropped during the course of the study.

Treatment Regimen - The regimen begins with a one week washout period. Each morning the subject is to wash her face with a suitable cleanser (*e.g.*, Olay Purifying Mud Lathering Cleanser, available from The Procter & Gamble Company, Cincinnati, OH), gently dry with a towel, apply a stock moisturizer (*e.g.*, Vehicle as described in Table 2 with 3% glycerine, no panthenol, and 0.3% disodium EDTA) to the appropriate side of the face, wait 5 minutes, and then apply a UV blocking lotion (*e.g.*, Olay Natural White UV Moisturizing Lotion SPF 15, available from The Procter & Gamble Company, Cincinnati, OH). Each night the subject is to wash her face with a suitable cleanser (*e.g.*, Olay Purifying Mud Lathering Cleanser, available from The Procter & Gamble Company, Cincinnati, OH), gently dry with a towel, and apply the stock moisturizer.

Each subject receives two coded test formulations for twice daily application to either the left or right side of the face. Each morning the subject is to wash her face with a suitable cleanser (*e.g.*, Olay Purifying Mud Lathering Cleanser, available from The Procter & Gamble Company, Cincinnati, OH), gently dry with a towel, apply the test formulation to the appropriate side of the face, wait 5 minutes, and then apply a UV blocking lotion (*e.g.*, Olay Natural White UV Moisturizing Lotion SPF 15, available from The Procter & Gamble Company, Cincinnati, OH). Each night the subject is to wash her face with a suitable cleanser (*e.g.*, Olay Purifying Mud Lathering Cleanser, available from The Procter & Gamble Company, Cincinnati, OH), gently dry with a towel, and apply the test formulation to the appropriate side of the face. Participants are to apply 0.5g of the appropriate test formulation on each side of the face. The test formulation should be applied with the fingers using gentle pressure and in a circular motion. Test formulations included a vehicle control, the vehicle + 1% Phlorogine, and the vehicle + 5% vitamin B3. These test formulas (not claimed) are set forth in Table 2.

Table 2

| | Vehicle | Vehicle + 1% Phlorogine | Vehicle + 5% Vitamin B3 | Vehicle + 0.4% Hexylresorcinol |
|---|---|---|---|---|
| Water | Q.S. | Q.S. | Q.S. | Q.S. |

(continued)

| | Vehicle | Vehicle + 1% Phlorogine | Vehicle + 5% Vitamin B3 | Vehicle + 0.4% Hexylresorcinol |
|---|---|---|---|---|
| Phlorogine | --- | 1.000 | --- | --- |
| Niacinamide | --- | --- | 5.0000 | --- |
| Hexylresorcinol | --- | --- | --- | 0.4000 |
| Glycerin | 10.0000 | 10.0000 | 10.0000 | 10.0000 |
| Isohexadecane | 3.0000 | 3.0000 | 3.0000 | 3.0000 |
| Polyacrylamide(and)C13-14 | 2.0000 | 2.0000 | 2.0000 | 2.0000 |
| Isoparaffin(and)Laureth-7 *A | | | | |
| Dimethicone and Dimethiconol *B | 2.0000 | 2.0000 | 2.0000 | 2.0000 |
| Isopropyl Isostearate | 1.3300 | 1.3300 | 1.3300 | 1.3300 |
| Tocopheryl Acetate | 0.5000 | 0.5000 | 0.5000 | 0.5000 |
| Panthenol | 1.0000 | 1.0000 | 1.0000 | 1.0000 |
| Cetyl Alcohol | 0.3200 | 0.3200 | 0.3200 | 0.3200 |
| Sucrose Polycottonseedate | 0.6700 | 0.6700 | 0.6700 | 0.6700 |
| Cetearyl Glucoside/Cetearyl Alcohol*C | 0.2000 | 0.2000 | 0.2000 | 0.2000 |
| Stearyl Alcohol | 0.4800 | 0.4800 | 0.4800 | 0.4800 |
| Behenyl Alcohol | 0.4000 | 0.4000 | 0.4000 | 0.4000 |
| Polymethylsilsesquioxane *D | 0.2500 | 0.2500 | 0.2500 | 0.2500 |
| Ethylparaben | 0.2000 | 0.2000 | 0.2000 | 0.2000 |
| Propylparaben | 0.1000 | 0.1000 | 0.1000 | 0.1000 |
| Disodium EDTA | 0.1000 | 0.1000 | 0.1000 | 0.1000 |
| Benzyl Alcohol | 0.4000 | 0.4000 | 0.4000 | 0.4000 |
| PEG-100 Stearate | 0.1000 | 0.1000 | 0.1000 | 0.1000 |
| *A - Sepigel 305, available from SEPPIC, France.<br>*B - Dow Corning 1503 Fluid, available from Dow Corning, Midland, MI.<br>*C - Emulglade PL 68/50, available from Cognis GmbH, Germany.<br>*D - Tospearl 2000, available from Momentive Performance Materials, Albany, NY. | | | | |

Images of the facial treatment sites are captured at baseline (week 0), and after 4 and 8 weeks of treatment and analyzed for changes to skin color and spot size and color. Prior to image collection the participant's face is washed with the above referenced cleanser and allowed to dry (approximately 20 minutes). Images are collected of the right and left side of the participant's face. Images are collected using a digital camera (*e.g.*, Fuji F2 Pro digital SLR) equipped with a suitable lens for facial imaging (*e.g.*, 60mm Nikor lens). Images are saved in a suitable file format such as RAW format at a suitable camera resolution. Lighting is provided by a flash source (*e.g.*, 1000W strobe with color temperature of about 5600K). The camera and lighting are equipped with polarizing filters to reduce specular reflection. A fixed color chart captured in each images allowing for computerized color calibration and correct of the study images.

The image file is processed via algorithms to yield a grayscale concentration map of eumelanin. The algorithm analyses every pixel of the RAW image and calculates the concentration of eumelanin present. A suitable algorithm involves decompiling the red, green, and blue values for every pixel. The RGB values for each pixel are processed and compared to known standards to yield the melanin concentration. The melanin concentration for each pixel is assigned a grey scale value from 0 to 255. Upon recombining the pixel array, a parametric grayscale concentration map of eumelanin (and/or other chromophores such as oxyhemoglobin) is produced. Figure 4 depicts a full color image of a participant as collected. Figure 5 depicts a melanin concentration map of the same participant.

Suitable methods for image collection and melanin mapping via RGB algorithms are described in U.S. Patent Application Publication Nos. 2008/0075340A1 to Cotton et al. (published March 27, 2008) and 2007/0161910A1 to Preece et al. (published July 12, 2007). A description of chromophore mapping can also be found in Matts, P.J., et al., "The Distribution of Melanin in Skin Determined In Vivo", British Journal of Dermatology, 156(4):620-628, April 2007.

Additional image analysis software, such as Optimas™ 6.5 (available from Media Cybernetics, Inc., Bethesda, MD), can be used to select a region of interest from the RAW image or from the resulting melanin map. A region of interest is selected to narrow the image to areas of the face where test formulation was targeted for application or to areas of the face of particular interest. For example, in the present study, a suitable region of interest of the facial skin surface includes the cheeks and periorbital region (*i.e.*, area bounded on one side approximately 1cm from hair line; bounded on the opposite side by a line parallel to the bridge of the nose along the upper edge and then along the nasolabial fold on the lower edge; bounded at the top along a line across the temple parallel to the upper eyelid and then approximately along lower edge of the orbital bone, and bounded at the bottom along a line parallel to the lower lip). The region of interest of the melanin map is further analyzed to calculate melanin spot area fraction and melanin evenness.

Another commercially available image collection and melanin mapping system is contact SIAscopy utilizing SIAscope V (available from Astron Clinica, Ltd., UK) or non-contact SIAscopy utilizing conventional digital imaging equipment (available from Astron Clinica, Ltd., UK). Another commercially available image collection and melanin mapping system is the VISIA® Complexion Analysis system utilizing the RBX™ technology (available from Canfield Scientific, Inc., Fairfield, NJ). The RBX™ technology transforms an image from RGB color into a RBX color-space where the red and brown channels represent hemoglobin and melanin distributions. The VISIA®/RBX™ is capable of spot area detection. *See* RBX™ Technology Overview White Paper, available at http://www.canfieldsci.com/FileLibrary/RBX%20tech%20overview-LoRz1.pdf or Canfield Imaging Systems, 253 Passaic Avenue, Fairfield, New Jersey 07004-2524.

Data from the melanin map are used to calculate Melanin Sport Area Fraction Percentage and Melanin Evenness. Melanin Spot Area Fraction Percentage (SAF) is calculated as the ratio of area occupied by melanin spots to the skin measurement area (*i.e.*, the region of interest) multiplied by 100. This percentage may be used to indicate size change of hyperpigmented areas. A lower percentage reflects smaller and/or fewer melanin spots.

Melanin Evenness is calculated as the standard deviation of the mean individual pixel grey scale value over the skin measurement area (*i.e.*, the region of interest). A lower value reflects more even melanin pigmentation. Further description of melanin spot area fraction and melanin evenness can also be found in Kinball, A.B., et al., "Reduction in the appearance of facial hyperpigmentation after use of moisturizers with a combination of topical niacinamide and N-acetyl glucosamine: results of a randomized, double-blind, vehicle-controlled study", British Journal of Dermatology, 162(2):435-441, February 2010.

Phlorogine was the best performer after 4 weeks, significantly ($p <= 0.10$) reducing hyperpigmented spots better than the control and the other test compositions. After 8 weeks, the vitamin B3 composition was the best performer significantly ($p <= 0.10$) reducing hyperpigmented spots better than the vehicle. Additionally, hexylresorcinol significantly ($p <= 0.10$) reduced hyperpigmented spots better than the vehicle as did Phlorogine.

Table 3 summarizes the image analysis data, wherein "SAF" is the mean Spot Area Fraction and "Δ SAF" is the mean change in Spot Area Fraction from an adjusted common baseline (week 0), and "Δ SAF test leg - Δ SAF vehicle" is the difference of the mean change in Spot Area Fraction of the test formulation minus the mean change in Spot Area Fraction of the vehicle.

Table 3

| | Week 0 | Week 4 | | | | Week 8 | | | |
|---|---|---|---|---|---|---|---|---|---|
| | SAF | SAF | Δ SAF | Δ SAF test leg - Δ SAF vehicle | P-Value*1 | SAF | Δ SAF | Δ SAF test leg - Δ SAF vehicle | P-Value*1 |
| Vehicle | 12.111*2 | 12.025 | -0.085 | --- | --- | 12.693 | 0.582 | --- | --- |
| 1% Phlorogine | | 11.749 | -0.361 | -0.276 | **0.0159** | 12.372 | 0.262 | -0.321 | **0.0064** |
| 5% Vitamin B3 | | 12.000 | -0.110 | -0.026 | 0.8189 | 12.281 | 0.171 | -0.412 | **0.0004** |
| 0.4% hexylresorcinol | | 11.898 | -0.213 | -0.128 | 0.2314 | 12.418 | 0.308 | -0.274 | **0.0125** |

*1 - Statistically significant Δ SAF test leg - Δ SAF vehicle values in boldface.
*2 - Adjusted Common Baseline

It is recognized that the SAF for the vehicle and the test legs increased from baseline (week 0) to Week 8. This test was performed in Beijing, China from approximately January 21 to approximately March 21. In this time period, the length of day increases by approximate 2.5 hours. While a UV product was used during the test to reduce the impact of seasonal skin darkening, it is believed that the SAF increase from baseline to Week 8 is attributable to seasonal skin darkening. Seasonal skin darkening is the natural darkening (*i.e.*, tanning) that occurs seasonally due to increased sunlight and UV exposure.

A separate study similar to the *in vivo* test described above was performed using a vehicle (similar to that described in Table 2), 5% niacinamide in vehicle, and 1% Sepiwhite (N-undecylenoyl-L-phenylalanine) in vehicle. Table 4 summarizes the image analysis data, wherein "SAF" is the mean Spot Area Fraction and "Δ SAF" is the mean change in Spot Area Fraction from an adjusted common baseline (week 0), and "Δ SAF test leg - Δ SAF vehicle" is the difference of the mean change in Spot Area Fraction of the test formulation minus the mean change in Spot Area Fraction of the vehicle.

Table 4

| | Week 0 | Week 4 | | | | Week 8 | | | |
|---|---|---|---|---|---|---|---|---|---|
| | SAF | SAF | Δ SAF | Δ SAF test leg - Δ SAF vehicle | P-Value*1 | SAF | Δ SAF | Δ SAF test leg - Δ SAF vehicle | P-Value*1 |
| Vehicle | 14.634*2 | 12.673 | -1.961 | --- | --- | 12.476 | -2.158 | --- | --- |
| 1% Sepiwhite | | 12.240 | -2.394 | -0.433 | **0.0003** | 11.888 | -2.746 | -0.588 | **<0.0001** |
| 5% Vitamin B3 | | 12.557 | -2.077 | -0.116 | 0.2936 | 12.169 | -2.465 | -0.307 | **0.0119** |
| *1 - Statistically significant (Δ SAF test leg - Δ SAF vehicle) values in boldface.<br>*2 - Adjusted Common Baseline | | | | | | | | | |

The Sepiwhite composition was the best performer after 4 weeks significantly ($p <= 0.05$) reducing hyperpigmented spots better than the control and the other test composition. After 8 weeks, the Sepiwhite composition was the best performer significantly ($p <= 0.05$) reducing hyperpigmented spots better than the vehicle. Likewise, vitamin B3 significantly ($p <= 0.05$) reduced hyperpigmented spots better than the vehicle.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A method of improving the appearance of a hyperpigmented spot on a facial skin surface comprising:

a. inspecting the facial skin surface for hyperpigmented spots in need of treatment based on size and/or color;
b. identifying a hyperpigmented spot on the facial skin surface in need of treatment; and
c. applying a composition to the hyperpigmented spot on a facial skin surface, the composition comprising:

i. a safe and effective amount of an fast-acting agent for hyperpigmented spots, wherein the fast-acting agent comprises *Laminaria Saccharina* extract, and
ii. a safe and effective amount of a slow-acting agent for hyperpigmented spots, wherein the slow-acting agent comprises vitamin B3;

wherein the composition is applied at least daily for a period of time sufficient for both the fast-acting agent and slow-acting agent to improve the appearance of the hyperpigmented spot.

**2.** The method according to claim 1, wherein the composition is applied at least once or twice a day for at least eight weeks.

**3.** The method according to any one of the preceding claims, wherein the improvement is a size reduction of the hyperpigmented spot, increased lightness of the hyperpigmented spot, or a reduction in melanin of the hyperpigmented spot.

**4.** The method according to any one of the preceding claims, wherein the first composition further comprises a sunscreen active, a skin tone agent, or combinations thereof.

**5.** The method according to any one of the preceding claims, wherein the first composition is locally applied to the hyperpigmented spot by an applicator, an applicator dosing between 1 to 50 uL/$cm^2$ of the first composition.

**6.** The method according to any one of the preceding claims further comprising the step of identifying a hyperpigmented spot on a skin surface, identifying a hyperpigmented spot by an imaging device.

**7.** The method according to any one of the preceding claims wherein the fast-acting agent further comprises N-undecylenoyl-L-phenylalanine.

**Patentansprüche**

**1.** Verfahren zur Verbesserung des Erscheinungsbilds eines hyperpigmentierten Flecks auf einer Gesichtshautoberfläche, umfassend:

a. Untersuchen der Gesichtshautoberfläche auf behandlungsbedürftige hyperpigmentierte Flecken basierend auf Größe und/oder Farbe;
b. Identifizieren eines hyperpigmentierten Flecks auf der behandlungsbedürftigen Gesichtshautoberfläche; und
c. Aufbringen einer Zusammensetzung auf den hyperpigmentierten Fleck auf einer Gesichtshautoberfläche, wobei die Zusammensetzung Folgendes umfasst:

i. eine sichere und wirksame Menge eines schnell wirkenden Mittels für hyperpigmentierte Flecken, wobei das schnell wirkende Mittel *Laminaria-Saccharina-Extrakt* umfasst, und
ii. eine sichere und wirksame Menge eines langsam wirkenden Mittels für hyperpigmentierte Flecken, wobei das langsam wirkende Mittel Vitamin B3 umfasst;

wobei die Zusammensetzung wenigstens täglich für einen Zeitraum aufgebracht wird, der dafür ausreicht, dass sowohl das schnell wirkende Mittel als auch das langsam wirkende Mittel das Erscheinungsbild des hyperpigmentierten Flecks verbessern.

**2.** Verfahren nach Anspruch 1, wobei die Zusammensetzung mindestens einmal oder zweimal täglich für mindestens acht Wochen aufgebracht wird.

**3.** Verfahren nach einem der vorstehenden Ansprüche, wobei die Verbesserung aus einer Verringerung der Größe des hyperpigmentierten Flecks, zunehmender Helligkeit des hyperpigmentierten Flecks oder einer Verringerung des Melanins des hyperpigmentierten Flecks besteht.

**4.** Verfahren nach einem der vorstehenden Ansprüche, wobei die erste Zusammensetzung ferner einen Sonnenschutzwirkstoff, ein Hauttonisierungsmittel oder Kombinationen davon umfasst.

**5.** Verfahren nach einem der vorstehenden Ansprüche, wobei die erste Zusammensetzung mittels eines Applikators, eines Applikators, der zwischen 1 bis 50 $\mu$l/$cm^2$ der ersten Zusammensetzung dosiert, lokal auf den hyperpigmentierten Fleck aufgebracht wird.

**6.** Verfahren nach einem der vorstehenden Ansprüche, ferner umfassend den Schritt des Identifizierens eines hyperpigmentierten Flecks auf einer Hautoberfläche, des Identifizierens eines hyperpigmentierten Flecks mittels einer Bildgebungsvorrichtung.

**7.** Verfahren nach einem der vorstehenden Ansprüche, wobei das schnell wirkende Mittel ferner N-Undecylenoyl-L-phenylalanin umfasst.

## Revendications

**1.** Procédé d'amélioration de l'apparence d'une tache hyperpigmentée sur une surface de peau du visage, comprenant :

a. l'inspection de la surface de peau du visage à la recherche de taches hyperpigmentées nécessitant un traitement sur base de la taille et/ou de la couleur,
b. l'identification d'une tache hyperpigmentée sur la surface de peau du visage nécessitant un traitement ; et
c. l'application d'une composition sur la tache hyperpigmentée sur une surface de peau du visage, la composition comprenant :

i. une quantité sûre et efficace d'un agent à action rapide pour taches hyperpigmentées, dans lequel l'agent à action rapide comprend un extrait de *Laminaria Saccharina,* et
ii. une quantité sûre et efficace d'un agent à action lente pour taches hyperpigmentées, dans lequel l'agent à action lente comprend de la vitamine B3 ;

dans lequel la composition est appliquée au moins quotidiennement pendant un laps de temps suffisant pour que tant l'agent à action rapide que l'agent à action lente améliorent l'apparence de la tache hyperpigmentée.

**2.** Procédé selon la revendication 1, dans lequel la composition est appliquée au moins une fois ou deux fois par jour pendant au moins huit semaines.

**3.** Procédé selon l'une quelconque des revendications précédentes, dans lequel l'amélioration est une réduction de taille de la tache hyperpigmentée, une clarté accrue de la tache hyperpigmentée, ou une réduction de mélanine de la tache hyperpigmentée.

**4.** Procédé selon l'une quelconque des revendications précédentes, dans lequel la première composition comprend en outre un agent actif contre les rayons solaires, un agent de teint de la peau, ou des combinaisons de ceux-ci.

**5.** Procédé selon l'une quelconque des revendications précédentes, dans lequel la première composition est appliquée localement sur la tache hyperpigmentée par un applicateur, un applicateur dosant entre environ 1 et environ 50 $\mu l/cm^2$ de la première composition.

**6.** Procédé selon l'une quelconque des revendications précédentes, comprenant en outre l'étape d'identification d'une tache hyperpigmentée sur une surface de peau, identifiant une tache hyperpigmentée par un dispositif d'imagerie.

**7.** Procédé selon l'une quelconque des revendications précédentes, dans lequel l'agent à action rapide comprend en outre de la N-undécylénoyl-L-phénylalanine.

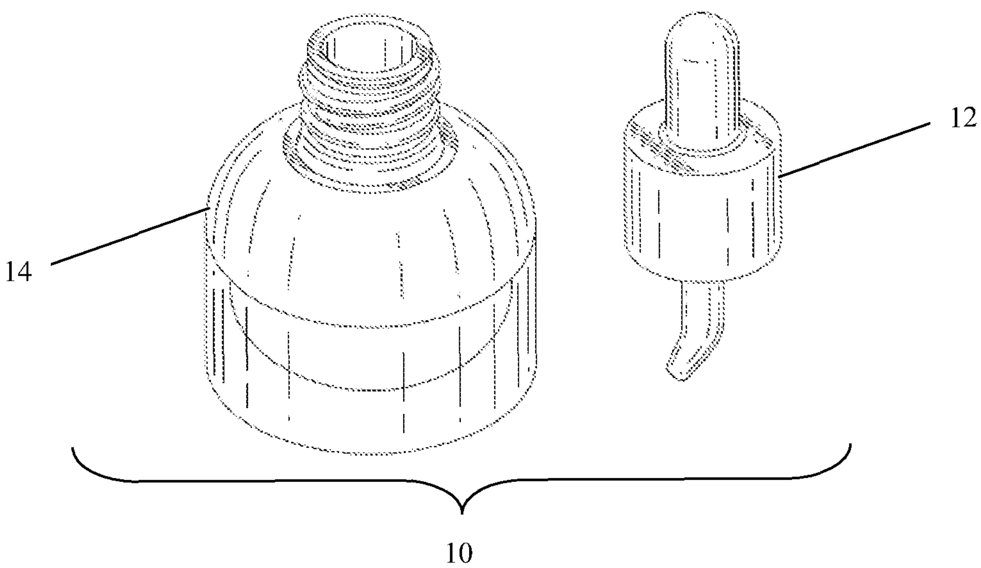


Fig. 1

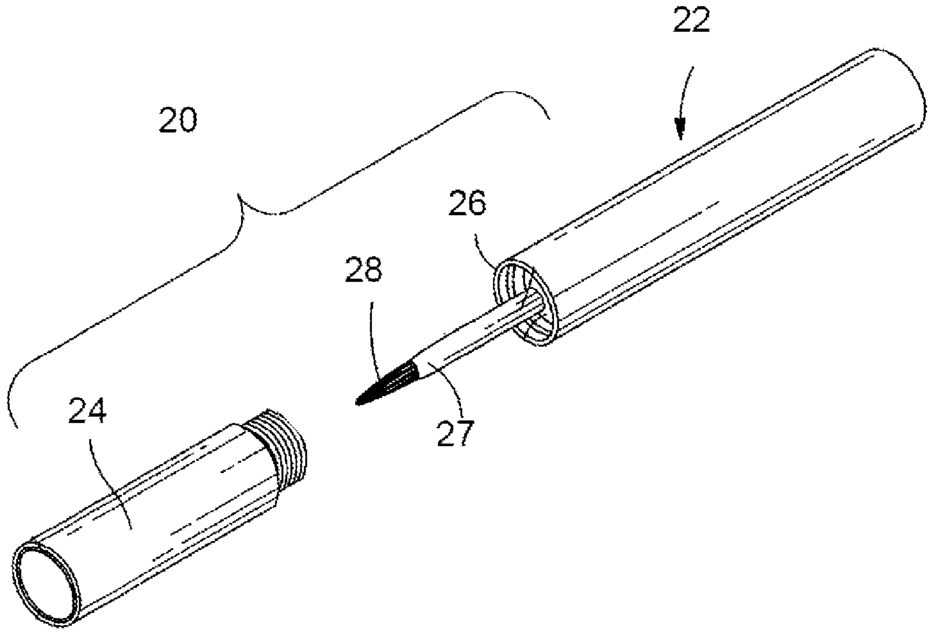


Fig. 2

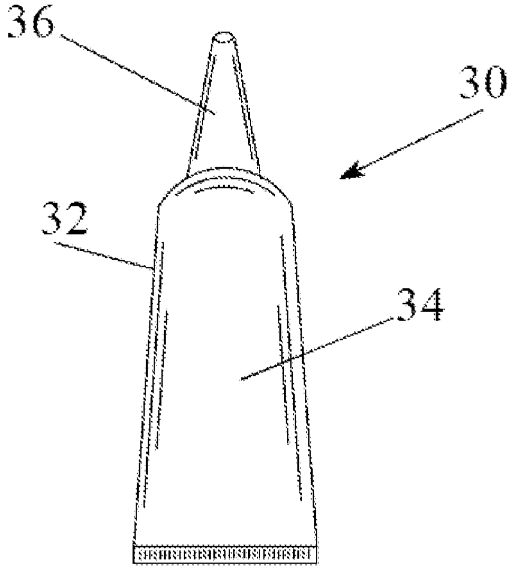


Fig. 3

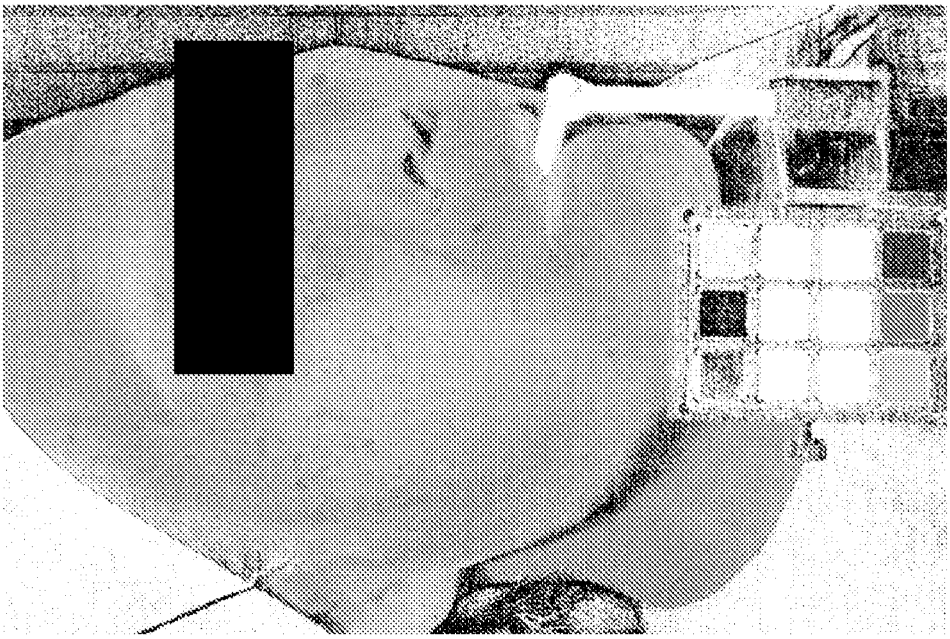

Fig. 5

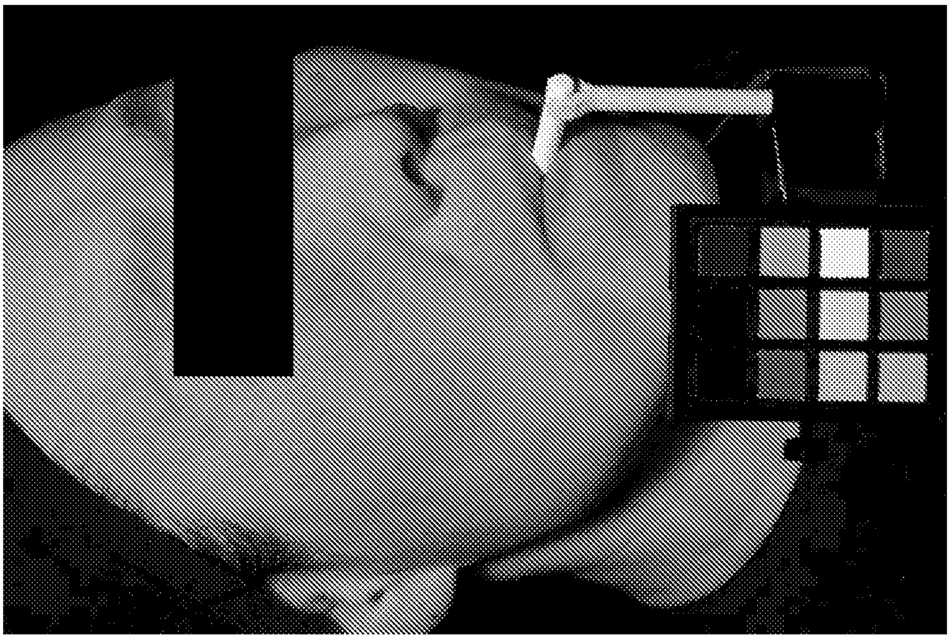

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080119527 A1
- EP 1074262 B1
- US 20060275237 A1
- US 20040175347 A1
- US 3755560 A
- US 4421769 A
- US 20080075340 A1, Cotton
- US 20070161910 A1, Preece


### Non-patent literature cited in the description


- **STRYER.** Biochemistry. W.H. Freeman and Company, 1981
- Personal Care Product Council's. International Cosmetic Ingredient Dictionary and Handbook
- The Personal Care Product Council's. International Cosmetic Ingredient Dictionary and Handbook
- McCutcheon's Detergents and Emulsifiers. 1986, 317-324
- **MATTS, P.J. et al.** The Distribution of Melanin in Skin Determined In Vivo. *British Journal of Dermatology,* April 2007, vol. 156 (4), 620-628
- **KINBALL, A.B. et al.** Reduction in the appearance of facial hyperpigmentation after use of moisturizers with a combination of topical niacinamide and N-acetyl glucosamine: results of a randomized, double-blind, vehicle-controlled study. *British Journal of Dermatology,* February 2010, vol. 162 (2), 435-441